# EUROPÄISCHE PATENTSCHRIFT

(11) **EP 1 174 420 B1**
(45) Veröffentlichungstag und Bekanntmachung des Hinweises auf die Patenterteilung: **06.10.2004**
(21) Anmeldenummer: 01115717.9
(22) Anmeldetag: 06.07.2001
(51) Int. Cl.: C07C 281/06

(54) **Verfahren zur Herstellung von Hydrazodicarbonamid (HDC) über Ketimine**
Process for the preparation of hydrazodicarbonamide (HDC) through ketimines
Procédé pour la préparation de hydrazodicarbonamide (HDC) en passant par une kétimine

(30) Priorität: 19.07.2000 DE 10035010
(43) Veröffentlichungstag der Anmeldung: 23.01.2002
(73) Patentinhaber: Bayer Chemicals AG, 51368 Leverkusen (DE)
(72) Erfinder: Jautelat, Manfred Dr., 51399 Burscheid (DE); Leidinger, Walter, Dr., 40597 Düsseldorf (DE)

(56) Entgegenhaltungen:
- DE-A- 2 803 614
- GB-A- 1 146 233
- ELVERS B. ET AL.: "Ullmann's Encyclopedia of Industrial Chemistry, 5th ed., Band A13" , ULLMANN'S ENCYCLOPEDIA OF INDUSTRIAL CHEMISTRY. HIGH-PERFORMANCE FIBERS TO IMIDAZOLE AND DERIVATIVES, WEINHEIM, VCH VERLAG, DE, SEITEN 179-186 XP002177765 * Seite 182 - Seite 185 *
- HAYASHI, HIROMU: "Hydrazine synthesis by a catalytic oxidation process" CATAL. REV. - SCI. ENG. ( 1990 ), 32(3), 229 -77 , XP001016066

## Beschreibung

Die vorliegende Erfindung betrifft ein neues Verfahren zur Herstellung von Hydrazodicarbonamid (HDC), das nach Oxidation zu Azodicarbonamid (ADC) als Polymerhilfsmittel (Ullmann's Encyclopedia of Industrial Chemistry, Vol. A13, 185) industriell Verwendung findet.

Es ist allgemein bekannt, dass HDC aus der Umsetzung von Hydrazin mit Harnstoff gewonnen wird (Ullmann's Encyclopedia of Industrial Chemistry, Vol. A13, 185). Die Ausbeuten bei diesem Verfahren sind gut. Allerdings ist Hydrazin nicht sehr preiswert, da es durch Chlorierung von Ammoniak hergestellt wird, und zudem als Gefahrstoff deklariert ist.

Weiterhin ist bekannt, dass man Hydrazin auch aus der Umsetzung von Ammoniak und Wasserstoffperoxid in Gegenwart von Ketonen und Katalysatoren erhalten kann. Doch die Ausbeuten lassen zu wünschen übrig. (Ullmann's Encyclopedia of Industrial Chemistry, Vol. A13, 182).

Aus DE-A-28 03 614 ist bekannt, dass man HDC durch Umsetzung eines Ketazins mit Harnstoff in wässrigem Medium unter Erwärmen herstellen kann. Wie das zu verwendende Ketazin hergestellt wird, wird jedoch nicht beschrieben.

Ferner ist bekannt, dass man Benzophenonimin mit Sauerstoff in Gegenwart von Katalysatoren zu Benzophenonazin oxidieren kann, das jedoch nur mit starken Säuren wie Schwefelsäure in Hydraziniumsulfat gespalten werden kann. (Catal. Rev. CR Sci. Eng., 1990, 32, 229-277). Die direkte Umsetzung dieses Benzophenonazins mit Harnstoff zu HDC gelingt aber nicht.

Es wurde nun gefunden, dass man Hydrazodicarbonamid (HDC) der Formel (I) 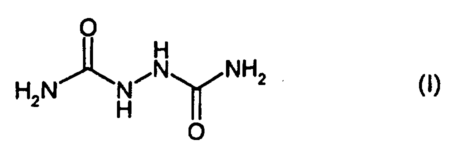 herstellen kann, indem man
a) aliphatische Ketone der Formel (II) 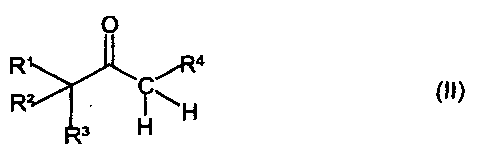 in welcher
   - R¹, R² und R³: gleich oder verschieden sind und für gegebenenfalls substituiertes Alkyl stehen und
   - R4: für Wasserstoff oder gegebenenfalls substituiertes Alkyl oder gegebenenfalls substituiertes Cycloalkyl steht oder
   - R¹ mit R² oder R⁴: gemeinsam eine Alkylenkette bildet,
   mit Ammoniak unter Druck zu Ketiminen der Formel (III) 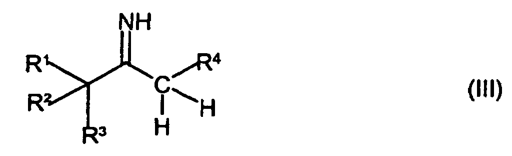 in welcher
   - R¹, R², R³ und R⁴: die oben angegebenen Bedeutungen haben,
   umsetzt,
b) diese Ketimine der Formel (III) mit Sauerstoff in Gegenwart von Katalysatoren zu Ketazinen der Formel (IV) 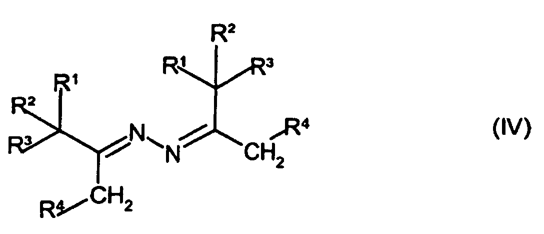 in welcher
   - R¹, R², R³ und R⁴: die oben angegebenen Bedeutungen haben, oxidiert und
c) diese Ketazine der Formel (IV) mit Harnstoff und Wasser in Gegenwart von Katalysatoren umsetzt.

Es ist als ausgesprochen überraschend zu bezeichnen, dass sich aliphatische Ketimine der Formel (III) in hoher Selektivität bilden und mit Sauerstoff zu Ketazinen der Formel (IV) in hoher Selektität oxidieren lassen. Es ist ebenfalls überraschend, dass sich die Ketazine der Formel (IV) mit Harnstoff und Wasser direkt zu Hydrazodicarbonamid (I) umsetzen lassen.

Das erfindungsgemäße Verfahren zeichnet sich durch eine Reihe von Vorteilen aus. So kann auf den Einsatz von Hydrazin verzichtet werden, als Oxidationsmittel wird Sauerstoff anstelle von Chlor verwendet, das eingesetzte Keton wird in der letzten Stufe frei gesetzt und kann in den Kreisprozess zurückgeführt werden. Durch diesen Kreisprozess ergibt sich summarisch die Oxidation von Harnstoff mit Sauerstoff zu Hydrazodicarbonamid und Wasser.

Verwendet man 3,3-Dimethyl-2-butanon (Pinakolon) als Einsatzketon, so kann der Kreisprozess des erfindungsgemäßen Verfahrens durch das Schema 1 veranschaulicht werden.

Die bei der Durchführung des erfindungsgemäßen Verfahrens verwendeten Ketone sind durch die Formel (II) allgemein definiert. Bevorzugt sind Verbindungen der Formel (II), in denen
- R¹, R² und R³: gleich oder verschieden sind und für geradkettiges oder verzweigtes Alkyl mit 1 bis 6 Kohlenstoffatomen stehen,
- R⁴: für Wasserstoff, für geradkettiges oder verzweigtes Alkyl mit 1 bis 6 Kohlenstoffatomen, oder für Cycloalkyl mit 3 bis 6 Kohlenstoffatomen steht, wobei diese Reste einfach bis zweifach, gleichartig oder verschieden substituiert sein können durch Halogen, Alkoxy mit 1 bis 4 Kohlenstoffatomen und/oder Cycloalkyl mit 3 bis 6 Kohlenstoffatomen, oder
- R¹ mit R² oder R⁴: eine Alkylenkette mit 2 bis 6 Kohlenstoffatomen bildet, wobei diese Kette einfach bis vierfach, gleichartig oder verschieden substituiert sein kann durch Halogen, Alkyl mit 1 bis 4 Kohlenstoffatomen und/oder Cycloalkyl mit 3 bis 6 Kohlenstoffatomen.

Besonders bevorzugt werden als Edukte Ketone der Formel (II) eingesetzt, in welcher
- R¹, R² und R³: gleich oder verschieden sind und für Methyl oder Ethyl stehen, und
- R⁴: für Wasserstoff, für Methyl, Ethyl, Isopropyl oder tert.-Butyl steht
oder
- R¹ mit R²: eine Alkylenkette mit 4 oder 5 Kohlenstoffatomen bildet oder
- R¹ mit R⁴: eine Alkylenkette mit 3 oder 4 Kohlenstoffatomen bildet.

Die Ketone der Formel (II) sind bekannt oder lassen sich nach bekannten Methoden herstellen.

Die Stufe a) des erfindungsgemäßen Verfahrens wird mit Ammoniak unter Druck (Eigendruck oder Inertgasdruck) von 20 bis 300 Hectopascal, vorzugsweise von 50 bis 200 Hectopascal durchgeführt. Ammoniak wird im molaren Verhältnis zum Keton von 1:1 bis 20:1, vorzugsweise 2:1 bis 10:1 eingesetzt. Zusätzlich kann gegebenenfalls ein Katalysator, vorzugsweise Ammoniumsalze wie Ammoniumchlorid oder Ammoniumsulfat, Verwendung finden.

Als Verdünnungsmittel kommen alle inerten Solventien in Betracht. Vorzugsweise wird ohne Verdünnungsmittel gearbeitet.

Die Reaktionstemperaturen bei der Durchführung der Stufe a) können in einem bestimmten Bereich variiert werden. Im allgemeinen arbeitet man bei Temperaturen zwischen 0°C und 200°C, vorzugsweise zwischen 20°C und 120°C.

Da das als Zwischenprodukt erhältliche Ketimin der Formel (III) leicht hydrolysiert, wird das Reaktionsgemisch vor Entspannung auf Temperaturen von 20°C bis -78°C, vorzugsweise auf 0°C bis -30°C abgekühlt. Die organische Phase wird von der wässrigen Phase getrennt und kann nach Trocknung aufgereinigt werden. Vorteilhaft wird das Gemisch aus Ketimin der Formel (III) und Edukt der Formel (II) ohne weitere Reinigung direkt in die Reaktionsstufe b) eingesetzt.

In die Reaktionsstufe b) wird das Ketimin der Formel (III) in reiner Form oder im Gemisch mit dem Keton der Formel (II) eingesetzt. Sauerstoff kann in reiner Form oder in Verdünnung mit inerten Gasen wie Stickstoff, vorzugsweise als Luft, unter Normaldruck oder unter Druck mit bis zu 20 Hectopascal verwendet werden.

Als Katalysatoren eignen sich Metallsalze der Metalle Cr, Mn, Fe, Co, Ni, Tl, Pb, Cu oder Ag, bevorzugt deren Halogenide, besonders bevorzugt Kupfer-Derivate wie beispielsweise CuCl oder CuBr. Diese können alleine, in wässriger Lösung, in Gemischen von verschiedenen Metallsalzen oder auf einem Träger aufgebracht eingesetzt werden. Im allgemeinen wird der Katalysator in Mengen von 0,01 bis 30 Gew.-%, vorzugsweise von 0,05 bis 20 Gew.-% verwendet.

Als Verdünnungsmittel kommen alle inerten Solventien in Betracht. Vorzugsweise wird mit nicht-umgesetztem Keton der Formel (II) aus der Reaktionsstufe b) gearbeitet.

Die Reaktionstemperaturen bei der Durchführung der Stufe b) können in einem bestimmten Bereich variiert werden. Im allgemeinen arbeitet man bei Temperaturen zwischen 0°C und 100°C, vorzugsweise zwischen 20°C und 80°C.

Um das bei der Oxidation gebildete Wasser zu binden, ist es vorteilhaft, ein Trockenmittel wie beispielsweise Zeolith oder Natriumsulfat zu verwenden.

Zur Aufarbeitung wird die organische Phase von der wässrigen Katalysatorphase getrennt und durch Destillation gereinigt. Das isolierte Keton der Formel (II) wird wieder in den Kreislauf zurückgeführt, die Katalysatorlösung wird direkt oder nach Aufarbeitung ebenfalls wieder verwendet und das Ketimin der Formel (IV) wird in die Stufe c) eingesetzt.

In der Reaktionsstufe c) des erfindungsgemäßen Verfahrens wird das Ketazin der Formel (IV) mit Harnstoff und Wasser in Gegenwart eines Katalysators umgesetzt. Ketazin und Harnstoff werden dabei im molaren Verhältnis 1:2 bis 1:5, vorzugsweise 1:2 bis 1:3 eingesetzt. Wasser wird equimolar oder im grossen Überschuss als Lösungsmittel verwendet.

Als Katalysatoren kommen Mineralsäuren wie beispielsweise Schwefelsäure oder Phosphorsäure, organische Säuren wie Trifluoressigsäure oder Trifluormethansulfonsäure oder saure Salze wie Ammoniumsulfat oder Ammoniumchlorid in Frage, die in katalytischen Mengen von 0,1 Gew.-% und mehr oder auch in grossem Überschuss eingesetzt werden können.

Als Verdünnungsmittel kommen polare Solventien wie Dimethylformamid (DMF), Essigsäure oder Wasser oder Gemische derselben in Betracht.

Die Reaktionstemperaturen bei der Durchführung der Stufe c) können in einem bestimmten Bereich variiert werden. Im allgemeinen arbeitet man bei Temperaturen zwischen 80°C und 150°c, vorzugsweise zwischen 100°C und 130°C.

Die Reaktion kann unter Normaldruck oder unter Druck ausgeführt werden, wobei die Entfernung des frei gesetzten Ammoniak gesichert sein muss.

Das Reaktionsprodukt Hydrazodicarbonamid der Formel (I) kann als Festprodukt durch Absaugen aus der Reaktionslösung isoliert werden. Das frei gesetzte Keton der Formel (II) wird aus der Reaktionslösung durch Destillation isoliert und in den Kreisprozess zurückgeführt.

Die Durchführung des erfindungsgemäßen Verfahrens wird durch die folgenden Beispiele veranschaulicht.

### Herstellungsbeispiel

**a) Pinakolonimin:** 50 g (0,5 mol) 3,3-Dimethyl-2-butanon (Pinakolon) und 0,5 g Ammoniumchlorid werden in einem 0,3 l Autoklaven vorgelegt, anschließend mit 100 ml (4 mol) flüssigem Ammoniak versetzt und auf 80°C erwärmt. Mit Stickstoff wird der Druck auf 200 bar erhöht. Dann wird der Ansatz 2 Stunden bei 80°C gerührt. Nach Abkühlen auf 0°C wird der Druckansatz langsam entspannt. Das Reaktionsgemisch wird in einen Scheidetrichter gegossen und die Phasen getrennt.
   - Wässrige Phase (unten, stark NH₃-haltig): 5,77 g
   - Organische Phase (oben): 49,29 g (0,3 mol, 60% Imin); GC: 37,0 % Keton u. 62,6 % Pinakolonimin; ¹H-NMR (CDCl₃, 400 MHz) δ: 1,15 (9H, s); 2,03 (3H, s); ∼9,0 (NH); GC/MS(CI): 100 (M+H⁺)
**b) Pinakolonazin:** 5,0 g einer Mischung aus Pinakolonimin (24,6 %, 12,4 mmol) in 3,3-Dimethyl-2-butanon (Pinakolon) werden mit 0,2 g Kupfer(I)-chlorid und 2,0 g Zeolith 134® bei Raumtemperatur vorgelegt. Unter Überleiten von Luft und Rühren wird anschließend 5 Stunden auf 40°C erhitzt. Nach dem Abkühlen wird das Reaktionsgemisch mit Dichlormethan verdünnt und mit verdünnter Ammoniaklösung ausgeschüttelt. Die organische Phase wird über Natriumsulfat getrocknet, filtriert und im Vakuum eingeengt. Man erhält 1,0 g (4,7 mmol, 76 % der Theorie) Pinakolonazin mit einem GC-Gehalt von 93,4 % und 2,5 % Pinakolon. ¹H-NMR (CDCl₃, 400 MHz) δ: 1,17 (18H, s); 1,68 (6H, s); GC/MS(EI): 196 (M⁺).
**c) Hydrazodicarbonamid (HDC)**: 1,96 g (10 mmol) Pinakolonazin werden mit 3,6 g (60 mmol) Harnstoff in 5 ml Wasser und 5 ml DMF unter Zusatz von 6,2 g Ammoniumsulfat übers Wochenende (72 h) unter Rückfluss erhitzt. Das ausgefallene Produkt wird abgesaugt, mit Wasser gewaschen und im Vakuum getrocknet. Man isoliert 1,0 g (8,5 mmol, 85 %) HDC vom F. 254° (Zers.).

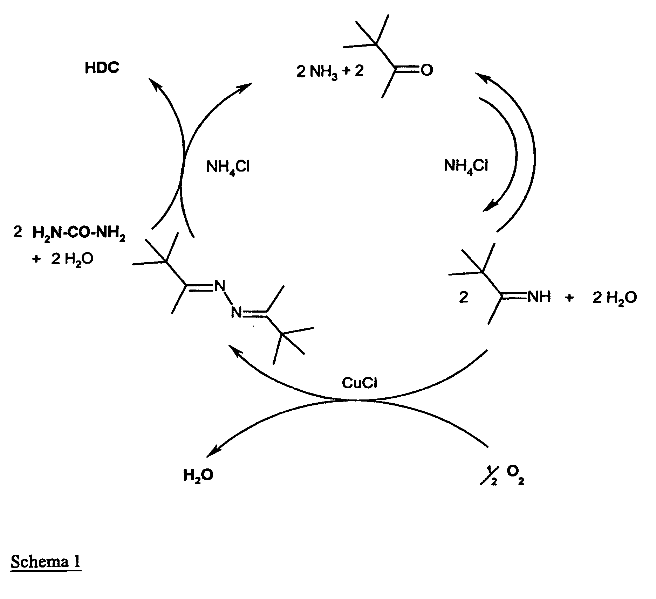

## Patentansprüche

1. Verfahren zur Herstellung von Hydrazodicarbonamid der Formel (I) 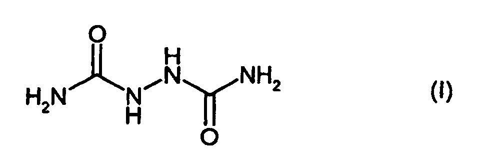 **dadurch gekennzeichnet, dass** man
a) aliphatische Ketone der Formel (II) 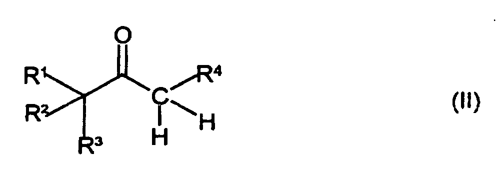 in welcher
R¹, R² und R³ gleich oder verschieden sind und für gegebenenfalls substituiertes Alkyl stehen und
R⁴ für Wasserstoff, gegebenenfalls substituiertes Alkyl oder gegebenenfalls substituiertes Cycloalkyl steht oder
R¹ mit R² oder R⁴ gemeinsam eine Alkylenkette bildet,
mit Ammoniak unter Druck zu Ketiminen der Formel (III) 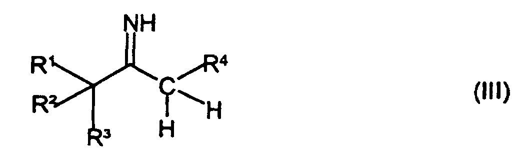 in welcher
R¹, R², R³ und R⁴ die oben angegebenen Bedeutungen haben, umsetzt,
b) diese Ketimine der Formel (III) mit Sauerstoff in Gegenwart von Katalysatoren zu Ketazinen der Formel (IV) 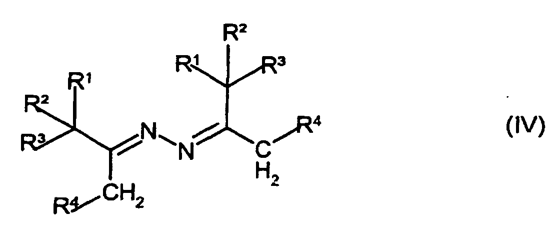 in welcher
R¹, R², R³ und R⁴ die oben angegebenen Bedeutungen haben, oxidiert und
c) diese Ketazine der Formel (IV) mit Harnstoff und Wasser in Gegenwart von Katalysatoren umsetzt.

2. Verfahren gemäß Anspruch 1, **dadurch gekennzeichnet, dass** Verbindungen der Formel (II) eingesetzt werden, in denen
R¹, R² und R³ gleich oder verschieden sind und für geradkettiges oder verzweigtes Alkyl mit 1 bis 6 Kohlenstoffatomen stehen,
R⁴ für Wasserstoff, für geradkettiges oder verzweigtes Alkyl mit 1 bis 6 Kohlenstoffatomen, oder für Cycloalkyl mit 3 bis 6 Kohlenstoffatomen steht, wobei dieser Rest einfach bis zweifach, gleichartig oder verschieden substituiert sein kann durch Halogen, Alkoxy mit 1 bis 4 Kohlenstoffatomen und/oder Cycloalkyl mit 3 bis 6 Kohlenstoffatomen,
oder
R¹ mit R² oder R⁴ eine Alkylenkette mit 2 bis 6 Kohlenstoffatomen bildet, wobei diese Kette einfach bis vierfach, gleichartig oder verschieden substituiert sein kann durch Halogen, Alkyl mit 1 bis 4 Kohlenstoffatomen und/oder Cycloalkyl mit 3 bis 6 Kohlenstoffatomen.

3. Verfahren gemäß Anspruch 1, **dadurch gekennzeichnet, dass** in der Stufe a) Ammoniak unter einem Druck von 20 bis 300 Hektopascal eingesetzt wird und eine Temperatur zwischen 0°C und 200°C als Reaktionstemperatur gewählt wird.

4. Verfahren gemäß Anspruch 1, **dadurch gekennzeichnet, dass** vor der Entspannung des Reaktionsgemisches dieses auf Temperaturen von 20°C bis -78°C abgekühlt wird.

5. Verfahren gemäß Anspruch 1, **dadurch gekennzeichnet, dass** in Reaktionsstufe b) Sauerstoff in reiner Form oder in Verdünnung mit inerten Gasen unter Normaldruck oder unter Druck mit bis zu 20 Hectopascal verwendet wird.

6. Verfahren gemäß Anspruch1, **dadurch gekennzeichnet, dass** Metallsalze der Metalle Cr, Mn, Fe, Co, Ni, Tl, Pb, Cu oder Ag als Katalysatoren eingesetzt werden.

7. Verfahren gemäß Anspruch 1, **dadurch gekennzeichnet, dass** die Reaktionstemperaturen in der Stufe b) zwischen 0°C und 100°C liegen.

8. Verfahren gemäß Anspruch 1, **dadurch gekennzeichnet, dass** in Stufe c) das Ketazin der Formel (IV) mit Harnstoff im molaren Verhältnis 1:2 bis 1:5 eingesetzt wird.

9. Verfahren gemäß Anspruch 1, **dadurch gekennzeichnet, dass** in Stufe c) Katalysatoren in Mengen von 0,1 Gew.-% und mehr oder auch in großem Überschuss eingesetzt werden.

10. Verfahren gemäß Anspruch 1, **dadurch gekennzeichnet, dass** in Stufe c) die Reaktionstemperaturen zwischen 80°C und 150°C liegen.

## Claims

1. Process for preparing hydrazodicarbonamide (HDC) of the formula (I) 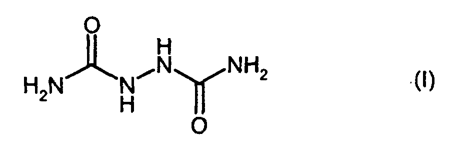 **characterized in that**
a) aliphatic ketones of the formula (II) 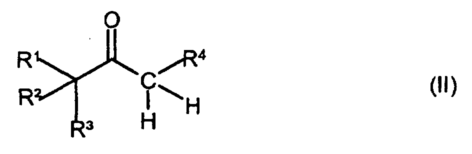 where
R¹, R² and R³ are identical or different and are each substituted or unsubstituted alkyl and
R⁴ is hydrogen or substituted or unsubstituted alkyl or substituted or unsubstituted cycloalkyl or
R¹ together with R² or R⁴ forms an alkylene chain,
are reacted with ammonia under pressure to form ketimines of the formula (III) 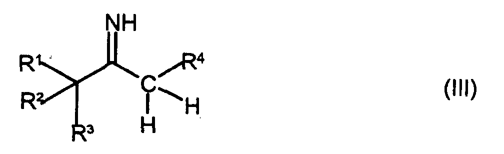 where
R¹, R², R³ and R⁴ are as defined above,
b) these ketimines of the formula (III) are oxidized by means of oxygen in the presence of catalysts to form ketazines of the formula (IV) 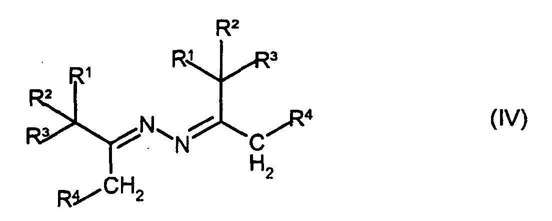 where
R¹, R², R³ and R⁴ are as defined above and
c) these ketazines of the formula (IV) are reacted with urea and water in the presence of catalysts.

2. Process according to Claim 1, **characterized in that** the compounds of the formula (II) used are ones in which
R¹, R² and R³ are identical or different and are each straight-chain or branched alkyl having from 1 to 6 carbon atoms,
R⁴ is hydrogen, straight-chain or branched alkyl having from 1 to 6 carbon atoms or cycloalkyl having from 3 to 6 carbon atoms, where this radical may bear one or two identical or different substituents selected from among halogen, alkoxy having from 1 to 4 carbon atoms and cycloalkyl having from 3 to 6 carbon atoms,
or
R¹ together with R² or R⁴ forms an alkylene chain having from 2 to 6 carbon atoms, where this chain may bear from 1 to 4 identical or different substituents selected from among halogen, alkyl having from 1 to 4 carbon atoms and cycloalkyl having from 3 to 6 carbon atoms.

3. Process according to Claim 1, **characterized in that** in step a) ammonia is used under a pressure of from 20 to 300 hectopascals and a temperature of from 0°C to 200°C is chosen as reaction temperature.

4. Process according to Claim 1, **characterized in that** the reaction mixture is cooled to temperatures of from 20°C to -78°C before depressurization.

5. Process according to Claim 1, **characterized in that** in reaction step b) oxygen is used in pure form or diluted with inert gases at atmospheric pressure or under a pressure of up to 20 hectopascals.

6. Process according to Claim 1, **characterized in that** salts of the metals Cr, Mn, Fe, Co, Ni, Tl, Pb, Cu or Ag are used as catalysts.

7. Process according to Claim 1, **characterized in that** the reaction temperatures in step b) are from 0°C to 100°C.

8. Process according to Claim 1, **characterized in that** in step c) the ketazine of the formula (IV) is used in a molar ratio to urea of from 1:2 to 1:5.

9. Process according to Claim 1, **characterized in that** in step c) catalysts are used in amounts of 0.1% by weight and more or in a large excess.

10. Process according to Claim 1, **characterized in that** the reaction temperatures in step c) are from 80°C to 150°C.

## Revendications

1. Procédé pour la préparation de l'hydrazodicarbonamide de formule (I) 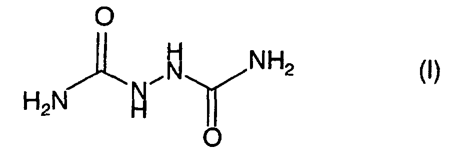 **caractérisé en ce que**
a) on convertit des cétones aliphatiques de formule (II) 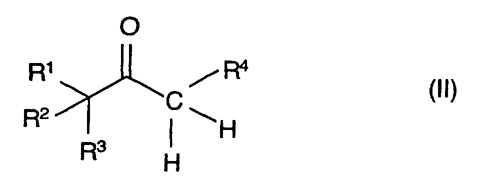 dans laquelle
R¹, R² et R³, ayant des significations identiques ou différentes, représentent chacun un groupe alkyle éventuellement substitué,
R⁴ représente l'hydrogène, un groupe alkyle éventuellement substitué ou un groupe cycloalkyle éventuellement substitué, ou bien
R¹ forme avec R² ou R⁴ une chaîne alkylène,
par réaction avec l'ammoniac sous pression, en cétimines de formule (III) 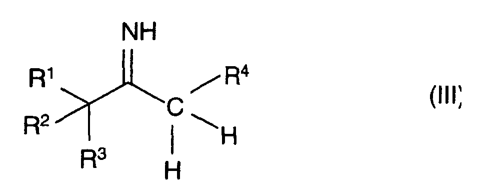
dans laquelle
R¹, R², R³ et R⁴ ont les significations indiquées ci-dessus,
b) on oxyde ces cétimines de formule (III) par l'oxygène en présence de catalyseurs, en cétazines de formule (IV) 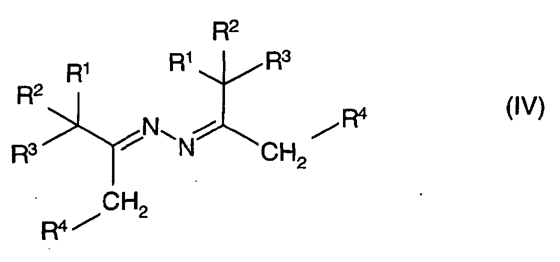 dans laquelle
R¹, R², R³ et R⁴ ont les significations indiquées ci-dessus, et
c) on fait réagir ces cétazines de formule (IV) avec l'urée et l'eau en présence de catalyseurs.

2. Procédé selon la revendication 1, **caractérisé en ce que** l'on met en oeuvre des composés de formule (II) dans laquelle
R¹, R² et R³ ayant des significations identiques ou différentes représentent chacun un groupe alkyle à chaîne droite ou ramifiée en C₁-C₆,
R⁴ représente l'hydrogène, un groupe alkyle à chaîne droite ou ramifiée en C₁-C₆ ou un groupe cycloalkyle en C₃-C₆, ces groupes pouvant porter 1 à 2 substituants identiques ou différents choisis parmi les halogènes, les groupes alcoxy en C₁-C₄ et/ou cycloalkyle en C₃-C₆,
ou bien
R¹ forme avec R² ou R⁴ une chaîne alkylène en C₂-C₆, cette chaîne pouvant porter 1 à 4 substituants identiques ou différents choisis parmi les halogènes, les groupes alkyle en C₁-C₄ et/ou cycloalkyle en C₃-C₆.

3. Procédé selon la revendication 1, **caractérisé en ce que**, au stade a), on utilise l'ammoniac sous une pression de 20 à 300 hectopascals et on observe une température de réaction allant de 0 à 200°C.

4. Procédé selon la revendication 1, **caractérisé en ce que**, avant détente du mélange de réaction, on le refroidit à une température de 20°C à -78°C.

5. Procédé selon la revendication 1, **caractérisé en ce que**, au stade de réaction b), on utilise l'oxygène à l'état pur ou dilué par un gaz inerte, à pression normale ou sous une pression pouvant aller jusqu'à 20 hectopascals.

6. Procédé selon la revendication 6, **caractérisé en ce que** l'on utilise en tant que catalyseurs des sels des métaux Cr, Mn, Fe, Co, Ni, Tl, Pb, Cu ou Ag.

7. Procédé selon la revendication 1, **caractérisé en ce que**, au stade b), la température de réaction va de 0 à 100°C.

8. Procédé selon la revendication 1, **caractérisé en ce que**, au stade c), la cétazine de formule (IV) et l'urée sont mises en oeuvre à un rapport molaire de 1:2 à 1:5.

9. Procédé selon la revendication 1, **caractérisé en ce que**, au stade c), on utilise les catalyseurs en quantité de 0,1 % en poids ou plus ou même en gros excès.

10. Procédé selon la revendication 1, **caractérisé en ce que**, au stade c), la température de réaction va de 80 à 150°C.
